# EUROPEAN PATENT APPLICATION

(11) **EP 2 149 375 A1**
(43) Date of publication of application: **03.02.2010**
(21) Application number: 08462004.6
(22) Date of filing: 28.07.2008
(51) Int. Cl.: A61K 33/06, A61K 33/12, A61P 17/00

(54) **Compositions for the treatment of dermatological diseases, and uses thereof**

(71) Applicant: Despharma Kft., 1124 Budapest (HU)
(72) Inventor: Feleki, Attila, 1138 Budapest (HU)
(74) Representative: Kovari, Zoltan

(57) **Abstract**

The present invention provides a composition containing
a) clay material with a negative surface charge;
b) magnesium ions either incorporated within the clay material and/or added separately;
c) water;
d) substantially non-cationic carrier;

where the total molar amount of magnesium ions compared to calcium ions is higher than 40% of the total molar amount of calcium ions.

The present invention further relates to the use of said composition for the preparation of a medicament for the treatment of skin diseases or skin conditions, where the formation and/ or recovery of epidermal barrier function has beneficial effects.

## Description

### Field of the Invention

The present invention relates to compositions for the treatment of dermatological diseases. In particular, the present invention relates to novel compositions for the treatment of dermatological diseases or skin conditions, where epidermal barrier formation and/or recovery would have beneficial effects. The invention further relates to the use of such compositions in the treatment of said diseases. The invention also relates to the use the compositions for the preparations of medicaments.

### Background of the Invention

The outermost layer of the skin is the epidermis which forms a barrier between the body and the external environment. This semipermeable epidermal barrier prevents both the escape of moisture and the entry of infectious or toxic substances. Common inflammatory skin disorders such as atopic dermatitis and psoriasis exhibit decreased barrier function. Improvement of epidermal barrier function is suggested to improve these skin disorders (The Journal of Clinical Investigation, 116(5): 1150-1158, 2006). In some hereditary diseases some major elements for intact barrier function are missing, like e.g. ceramides in case of atopic dermatitis.

Ion gradients of ions such as calcium and magnesium in the epidermis play a crucial role in skin barrier homeostasis. Topical application of calcium solution delayed barrier repair after barrier disruption, however, the application of magnesium salts accelerated barrier recovery (The Journal of Clinical Investigation, 89: 530-538, 1992; Arch. Dermarol. Res., 291:560-563, 1999). Normal skin surface has a negative potential against the inside and the potential decreases immediately after barrier disruption (J. Invest Dermatol., 69:324-327, 1977). Application of external electric potential without topical application of any bioactive chemical materials was reported to improve cutaneous barrier function (J. Invest Dermatol., 118:65-72, 2002).

The effects of topical application of ionic polymers on the damaged skin barrier was evaluated. Application of a nonionic polymer did not affect barrier recovery. Application of sodium salts of anionic polymers accelerated barrier recovery, while that of cationic polymers delayed it. Topical application of a sodium-exchange resin accelerated barrier recovery, but application of a calcium-exchange resin had no effect when the resins had the same structure. (Skin Pharmacol. Physiol., 18:36-41, 2005) Here, according to the authors' conclusion anionic polymer together with magnesium chloride did not support barrier recovery.

Several different treatments of inflammatory skin diseases are known. For example:
i) W00185163 suggests either cetyl myristate or cetyl myristate and cetyl palmitate for the treatment or prophylaxis of eczema and/or psoriasis;
ii) DE102007030198 suggests colloidal silica for the treatment of several skin diseases, like atopic dermatitis, psoriasis, inflammation, etc. Preferred embodiments also contain zeolites, like clinoptilolite, fausit, modenit, furthermore calcium carbonate, magnesium carbonate and water; and
iii) W006108414 discloses an agent for use as an oral or topical agents in the therapy and prophylaxis of skin diseases, especially psoriasis, neurodermitis, or atopic dermatitis, where the agent comprises zeolites and stinging nettle leaf extracts or calcium and/or magnesium salts.

Inflammatory skin diseases are sometimes associated with bacterial infections. E.g. *Staphylococcus aureus* superantigens are known to be present in more than 50% of patients with atopic dermatitis and psoriasis, and the severity of these diseases significantly correlated to enterotoxin production of the isolated *Staphylococcus aureus* strains (Journal of the American Academy of Dermatology, 53:67-72, 2005). Here we have to mention that atopic dermatitis is a hereditary skin symptom, which can be considered as a disease of primary barrier failure.

US patent No. 6,551,607 describes a method for sequestration of skin irritants, like superantigens (such as produced by the bacterium *Staphylococcus aureus*), cytokines, etc. Here, sequestering agent can be adsorbent clays, silicas, etc.

US5900258 describes compositions containing zeolites for preventing microorganisms from growing in a diaper, clothing, bedding, on the skin, etc. This invention was based on the finding according to which the microbial growth is inhibited by zeolites.

Although the role of the epidermal barrier was recognized in several skin diseases/ disorders, according to the state of the art there is no pharmaceutical/cosmetic composition available that were optimized to the finding according to which these skin diseases could be treated by epidermal skin barrier formation and/or recovery.

### Summary of the Invention

The present invention was made in view of the prior art described above, and the object of the present invention is to provide a pharmaceutically and cosmoceutically acceptable composition for the treatment of dermatological diseases, where epidermal barrier formation and/or recovery has beneficial effects.

To solve the problem, the present invention provides a composition containing
a) clay material with a negative surface charge;
b) magnesium ions either incorporated within the clay material and/or added separately;
c) water;
d) substantially non-cationic carrier;
   where the total molar amount of magnesium ions compared to calcium ions is higher than 40% of the total molar amount of calcium ions.

In a preferred embodiment the clay material is a zeolite, more preferably clinoptilolite.

In an other preferred embodiment the total molar amount of magnesium ions compared to calcium ions is higher than 90% of the total molar amount of calcium ions.

In an other preferred embodiment, the mean particle size of the clay material is between 1 nm and 100 µm, more preferably between 50 nm and 500 nm.

According to yet an other preferred embodiment the separately added magnesium ions are in the form of magnesium chloride or magnesium bromide, preferably magnesium chloride.

Yet according to an other embodiment, the invention relates to the use of the composition according to the invention for the preparation of a medicament /medical device for the treatment of skin diseases or skin conditions, where the formation and/or recovery of epidermal barrier function has beneficial effects.

According to a preferred embodiment, the skin diseases and skin conditions are selected from inflammatory skin diseases, increased fibroblast proliferation, pruritus, physical damage of the skin surface, xerosis, bruises, hyperproliferation states of the skin, and transepidermal water loss. According to a more preferred embodiment, the inflammatory skin disease is psoriasis, atopic dermatitis, or various types of eczema.

Since some of the skin disorders are not recognized as diseases, but rather as cosmetic problems, the composition according to the present invention is useful also in cosmetology, not only in dermatology. In other words, the term "medicament" in the present specification shall be understood beside being a product useful in medicine, also as a product useful in cosmetology.

According to an other preferred embodiment, the medicament is in the form of a cream, lotion, gel, ointment, cutaneous solution, suspension, spray, foam, bath additive, collodion, impregnated dressing or medicated plaster.

We have surprisingly found that a composition that is able to form an electric double layer in aqueous medium, and shows a stable negative charge, has a cation exchange capacity and cation binding capacity, furthermore contains total magnesium ions of at least 40% of the amount of calcium ions, in combination with a non-ionic, anionic, amphoteric or very weakly cationic carrier, is able to recover damaged epidermal barrier upon topical application on the affected skin area.

A negative charge can be formed by any natural or artificial clay material with a negative surface charge. Zeolites, especially clinoptilolite are the preferred choice of clay material according to the present invention. The clay material is preferably in the form of small particles (nanoparticles), since this average size range provides acceptable small particles to be applicable on skin surface, and acceptable high specific surface and charge.

Magnesium ions play a crucial role in the epidermal barrier function, however, calcium ions are found to be against its proper function. Accordingly, it is necessary to include a calculated amount of magnesium ions compared to calcium ions being present in the composition according to the present invention. The molar amount of magnesium ion to calcium ion ratio should be higher than 0.4, preferably higher than 0.9. Most preferably there are more magnesium ions in the composition than calcium ions. Magnesium ions can be introduced into the composition according to the invention either as part of the applied clay material, and/or separately, as any physiologically acceptable magnesium salt.

According to the present invention, the composition should be able to form an electric double layer in aqueous medium, and should provide a stable negative charge. Accordingly, the composition contains also water for providing said aqueous medium.

The composition according to the present invention also necessarily contains a substantially non-cationic, i.e. non-ionic, anionic, amphoteric or very weakly cationic carrier. Since the present invention aimed at providing a composition forming a negative net charge, only substantially non-cationic carriers could be acceptable. On the other hand compositions containing slightly cationic carriers are also within the scope of the present invention, if the total net charge of the composition remains negative.

We have found that the above detailed composition according to the present invention is useful for preparation of medicaments/medical devices for treating such diseases, where the formation and/or recovery of epidermal barrier function has beneficial effects, since these medicaments/medical devices improve disrupted epidermal barrier function. (The term "medical device" within the present description means a pharmaceutical or cosmetical product where not pharmacological but biophysical effect is thought to be the basis of the effectiveness of the product.) Several skin diseases are known to be associated with malfunction of the epidermal barrier, e.g. inflammatory skin diseases (like psoriasis, atopic dermatitis, or various types of eczema), increased fibroblast proliferation, pruritus, physical damage of the skin surface, xerosis (hyperproliferated scaly skin caused by dry enviroment), bruises, hyperproliferation states of the skin, and transepidermal water loss.

Since recovery of the damaged epidermal barrier is a longer process, it is necessary that the medicament of the present invention remains on the affected skin area as long as possible. Therefore, the form of the medicament according to the present invention should be able to keep the medicament on the skin surface for a longer time. Accordingly, cream, lotion, gel, ointment, cutaneous solution, suspension, spray, foam, bath additive, collodion, impregnated dressing or medicated plaster are preferable from this point of view. Soaps or shampoos are less preferred, however, are also within the scope of the present invention.

### Detailed Description of the Invention

Magnesium ions (in the form of MgCl₂) are historically documented having effect on regulation of fibroblast replication, barrier recovery acceleration, supressing effect on skin's Langerhans cells, which leads to lowering antigen presentation capacity of said cells.

Clinoptilolite (Ca-K-Mg-Na-zeolite), one of the most preferred zeolite according to the present invention, dispersed in water, show a strong negative surface charge, that is formed as an electric double layer around the clay material particles, which bind cations. Accordingly, a strong negative charge is formed in the dispersion medium. Zeta potential measurements were used for quantification of the magnitude of the electrical charge at the double layer. Any natural or artificial clay material with a negative surface charge is useful as the clay material according to the present invention, for example, bentonite, montmorillonite, beidelite, hectorite, saponite, stevensite, mordenit, laumonite, phillipsit, heulandit, stilbit, kabazit, stellerit, or laponite.

The clay material, especially the zeolite has the following beneficial effects regarding epidermal barrier formation and/or recovery:
i) the boost of negative electric charge;
ii) the cation exchange; and
iii) the cation binding capacity.
These three effects trigger the epidermal barrier formation and/or recovery.

One of the main advantage of using zeolites, especially clinoptilolite in the composition according to the invention is that it shows a synergic effect together with magnesium ions. Namely, binding Mg²⁺ ions by the clinoptilolite is increasing the bioavailability of magnesium ions by the skin.

However, we found that using zeolites and magnesium salts exclusively, will not lead to epidermal barrier recovery effects. But when using also a humectant (a hygroscopic substance, increasing water content capacity of the skin) rich emollient as said carrier, including water, to moisturize the skin, this will strongly initiate barrier recovery and prevent transepidermal water loss.

Accordingly, when using the above components, namely a clay material, preferably zeolite, more preferably clinoptilolite, furthermore magnesium ions, water and a substantially non-cationic carrier in a single composition, a surprisingly increased epidermal barrier recovery effect could be observed, because of the improvment/ formation of the stable negative potential on the affected skin surface, furthermore because of the cation exchange and cation binding capacity, magnesium supply and moisturizing of the skin occurs at the same time, all these effects support epidermal barrier recovery, made by one single composition according to the present invention.

The water content of the composition is essential regarding its efficacy. Besides its role in forming the aqueous medium with a negative net charge, water has also a role in dissolving added magnesium salts. The water content of the composition is between 1 and 90%, preferably between 30 and 80%.

We identified an additional beneficial effect of the composition of the present invention. Namely, a negative charge and the clay material together is able to eliminate positively charged superantigens, or even bacteria from the skin. Since some of the skin disorders are associated with bacterial infections (see above) this effect also increases the efficacy of the composition in the treatment of some of said skin diseases.

Any magnesium salt that are physiologically acceptable and does not impair negative zeta potential of the composition can be used. Both inorganic, or organic magnesium salts could be acceptable, namely magnesium salts of chloride, bromide, sulphate, phosphate, acetate, citrate, etc. Preferred magnesium salt is magnesium chloride or magnesium bromide. It is obvious for the person skilled in the art that magnesium salts can be used in the form of various hydrates. According to a most preferred embodiment of the present invention magnesium chloride hexahydrate is used as separately added magnesium source of the composition. The amount of the magnesium chloride hexahydrate can vary from about 0,02 w/w% to about 90 w/w%, preferably from about 0,2 w/w% to about 30 w/w% of the composition.

The composition of the present invention can also contain pharmaceutically acceptable additives. For example vitamins, especially dermatologically preferred vitamins could be added to the composition. Preservatives (e.g. methylparaben, propylparaben, butylparaben, diazolidinyl urea, sorbic acid, etc.), pH adjusting additives (sodium hydroxide, hydrogen chloride), moisturizers (e.g. urea, gold colloids, silver colloids, etc), pigments (iron oxide) that are known according to the state of the art can also be applied. Other pharmaceutically acceptable additives can also be applied according to the present invention, like glyceryl monostearate, cocoglycerides, glyceryl stearates, cetyl alcohol, isopropyl myristate, ceteareth-20, ceteareth-12, cetyl palmitate, etc.

The composition can also contain other active ingredients that are useful in the treatment of the dermatological disease or skin conditions to be treated. For example ceramide, ceramidase antagonists, or ceramide production agonists can be added to the composition in the treatment of atopic dermatitis, since this disease is characterized by low ceramide levels. For the treatment of psoriasis the addition of vitamin D analogs and/or steroids to the composition could be advantageous. Other examples for active ingredients in compositions for the treatment of psoriasis can include tar products and natural herbal extracts, like e.g. calendula. For the treatment of different types eczema the addition of various types of peptides could be useful. Also wound healing additives are useful in some of the above mentioned disease, here zinc-hyaluronate, silver or gold colloides are worth mentioning. Regulators of the inflammatory cascade elements which can effect barrier damage recovery (like e.g. interleukins 4, 8 or 10) can also be applied as additional active ingredients.

The optimal molar amount of the applied magnesium ions is dependent on the skin disorder to be treated. Namely, composition for the treatment of atopic dermatitis have lower amount of magnesium ions, since the treatment of an open wound on children can not be treated with compositions with high salt-contents because of irritation. However, compositions for the treatment of psoriasis should have higher magnesium ion concentration.

As mentioned before, the form of the medicament according to the present invention can be any formula able to keep the medicament on the skin surface for a longer time. Accordingly, cream, gel, lotion , ointment, cutaneous solution, suspension, spray, foam, bath additive, collodion, impregnated dressing or medicated plaster are preferred formulas.

Cream could be either oil-in-water or water-in-oil type. According to the present invention oil-in-water type cream is preferred. Several emulgators are acceptable for the purpose of the present invention, like e.g. alkyl sulphates, alkyl amines, alkyl pyrimidin compounds, etc.
Also there are several acceptable oils for cream formulation, like e.g. white petrolatum, paraffin, cetearyl alcohol, cocoglycerides, cetyl alcohol, isopropyl miristate, cetyl palmitate, butyrum cacao, oleum helianthi, cera alba, lanolin, isopropyl palmitate, stearic acid, magnesium stearate.

For preparation of a gel, the following gel forming additives could be used for example: cellulose gum (carboxymethyl cellulose), hydroxypropyl cellulose, methylcellulose, hydroxyethyl cellulose, ethylhydroxy cellulose, or laponite. Solvent/ dispergating medium for a gel is preferably a water/ glycerol or water proylenglycol mixture.

Zeta potential shows the inherent electric characteristic of the composition according to the invention. Briefly, zeta potential is the electrokinetic potential in colloidal systems, namely zeta potential is electric potential in the interfacial double layer at the location of the slipping plane versus a point in the bulk fluid away from the interface, i.e. the potential difference between the dispersion medium and the stationary layer of fluid attached to the dispersed particle. Since the healthy skin surface has a negative charge, the composition according to the invention shall be able to form an even more negative environment on the surface of the skin,a negative charge boost to trigger barrier repair cascading mechanisms. For this purpose, the composition should show a stable negative zeta potential, which can be measured during manufacture of the medicament of the present invention.

### Examples

Hereinafter, the present invention is described in more detail and specifically with reference to the Examples, which however are not intended to limit the present invention.

### Example 1a: Cream formulation for the treatment of atopic dermatitis

A cream was formulated for the treatment of atopic dermatitis, where said cream consisted of the following ingredients (INCI (International Nomenclature of Cosmetic Ingredients) names of the ingredients are provided) :

| **Ingredient** | **m/m %** |
|---|---|
| Aqua | 57.600 |
| White petrolatum | 17.153 |
| Cetearyl alcohol | 10.292 |
| Paraffin | 3.430 |
| Polysorbate 60 | 3.430 |
| Clinoptilolite | 3.439 |
| Glycerine | 2.579 |
| Alcohol | 1.543 |
| Magnesium chloride | 0.214 |
| Methylparaben | 0.171 |
| Phospholipid | 0.133 |
| Sphyngolipid | 0.016 |

### Example 1b: Zeta potential measurement

Zeta potential of the cream according to Example 1a was measured. Measurement was performed by Brookhaven ZetaPALS instrument in the presence of 1 mM MgCl₂ inert electrolyte at 30 °C. The instrument is equipped with Peltier element for the active temperature control. The ZetaPALS determines zeta potential using Phase Analysis Light Scattering method based on the shifted frequency spectrum. Palladium electrodes with acrylic supports were used for the measurement.

### Results of the zeta potential measurement:

Conductivity: 566 µS
Electroforetic mobility: -0.87* 10⁻⁸m²/Vs
Zeta potential (calculated by Smoluchowsky theory): -10.02 mV
Zeta potential (calculated by Hückel theory): -15.04 mV

Accordingly, zeta potential of the composition is within the required negative range.

### Example 1c: Electric surface potential measurements

Electric surface potential measurements were carried out with the cream of Example 1a on intact skin surfaces of human volunteers.

### Measurement conditions:

- Machine:: Consort C831 electrochemical analysator
- Electrodes:: EKG adhesive electrodes
- Connecting medium:: REXTRA EKG, EEG, EMG diagnostic gel for medical use, pH 6.5 Ingredients: Aqua, Carbomer, Triethanolamine, Methylchloroisothiazolinone, methylisothiazolinone
- Measured data row:: 25 points, 10 sec / point
- Place of measurements:: lower arm flexor area in inactive position
- Cream applied on:: a 5 x 5 cm surface, after 15 minutes cream removed
- Distance of the electrodes:: 10 cm
- Number of volunteers:: 3

Results are shown in the following Table 1.

**Table 1: Results of the electric surface potential measurements of Example 1c**

| | | **Volunteer 1** | | | **Volunteer 2** | | | **Volunteer 3** | | |
|---|---|---|---|---|---|---|---|---|---|---|
| **Meas. point** | **sec** | **M1** | **M2** | **result** | **M1** | **M2** | **result** | **M1** | **M2** | **result** |
| 1 | 0 | -65 | -122 | **-57** | 20 | -86 | **-106** | 33 | -98 | **-131** |
| 2 | 10 | -60 | -127 | **-67** | 20 | -90 | **-110** | 41 | -99 | **-140** |
| 3 | 20 | -55 | -133 | **-78** | 21 | -94 | **-115** | 43 | -101 | **-144** |
| 4 | 30 | -53 | -138 | **-85** | 28 | -98 | **-126** | 45 | -103 | **-148** |
| 5 | 40 | -52 | -145 | **-93** | 21 | -102 | **-123** | 45 | -105 | **-150** |
| 6 | 50 | -51 | -151 | **-100** | 21 | -108 | **-129** | 44 | -108 | **-152** |
| 7 | 60 | -47 | -158 | **-111** | 22 | -110 | **-132** | 45 | -11 | **-56** |
| 8 | 70 | -40 | -165 | **-125** | 22 | -114 | **-136** | 44 | -114 | **-158** |
| 9 | 80 | -36 | -172 | **-136** | 23 | -117 | **-140** | 45 | -118 | **-163** |
| 10 | 90 | -34 | -178 | **-144** | 15 | -121 | **-136** | 46 | -122 | **-168** |
| 11 | 100 | -31 | -183 | **-152** | 21 | -125 | **-146** | 45 | -128 | **-173** |
| 12 | 110 | -28 | -188 | **-160** | 22 | -129 | **-151** | 46 | -129 | **-175** |
| 13 | 120 | -21 | -192 | **-171** | 23 | -133 | **-156** | 46 | -133 | **-179** |
| 14 | 130 | -18 | -196 | **-178** | 23 | -138 | **-161** | 47 | -136 | **-183** |
| 15 | 140 | -17 | -199 | **-182** | 23 | -143 | **-166** | 48 | -139 | **-187** |
| 16 | 150 | -17 | -203 | **-186** | 25 | -146 | **-171** | 49 | -143 | **-192** |
| 17 | 160 | -16 | -205 | **-189** | 23 | -150 | **-173** | 49 | -145 | **-194** |
| 18 | 170 | -17 | -208 | **-191** | 23 | -153 | **-176** | 49 | -147 | **-196** |
| 19 | 180 | -16 | -211 | **-195** | 24 | -157 | **-181** | 50 | -149 | **-199** |
| 20 | 190 | -15 | -213 | **-198** | 23 | -160 | **-183** | 50 | -152 | **-202** |
| | | | | | | | | | | |
| | | -34 | -174 | **-140** | 22 | -124 | **-146** | 46 | -119 | **-165** |
| | | | | | | | | | | |
| avarage | | **M1** | **M2** | **result** | | | | | | |
| | | 11 | -139 | **-150 mV** | | | | | | |

where
- M 1:: potential difference between 2 points with connecting medium;
- M2:: potential different between 2 points with connecting medium and cream of Example 1a;
- Result:: surface electric potential difference between M1 and M2 in mV.

These results show a definite effect on the electric potential of the skin surface.

### Example 2: Cream formulation for the treatment of psoriasis

A cream was formulated for the treatment of psoriasis, where said cream consisted of the following ingredients (INCI (International Nomenclature of Cosmetic Ingredients) names of the ingredients are provided):

| **Ingredient** | **m/m %** |
|---|---|
| Aqua | 41.81 |
| Magnesium Chloride | 17.22 |
| White petrolatum | 14.22 |
| Cetearyl Alcohol | 8.59 |
| Zeolite* | 8.00 |
| Paraffin | 2.87 |
| Polysorbate 60 | 2.86 |
| Gylcerin | 3.00 |
| Alcohol | 1.29 |
| Methylparaben | 0.14 |

| | |
|---|---|
| *zeolite stands for clinoptilolite | |

### Example 3: Cream formulation for the treatment of psoriasis

A cream was formulated for the treatment of psoriasis on the scalp, where said cream consisted of the following ingredients (INCI (International Nomenclature of Cosmetic Ingredients) names of the ingredients are provided) :

| **Ingredient** | **m/m %** |
|---|---|
| Aqua | 86.86 |
| Magnesium Chloride | 4.00 |
| Glycerin | 4.95 |
| Cellulose Gum | 1.19 |
| Zeolite* | 2.00 |
| Propylene Glycol | 0.55 |
| Diazolidinyl Urea | 0.30 |
| Methylparaben | 0.12 |
| Propylparaben | 0.03 |

| | |
|---|---|
| *zeolite stands for clinoptilolite | |

### Example 4: Gel formulation

A gel containing the composition of the present invention was formulated with the following ingredients:

| **Ingredient** | **m/m %** |
|---|---|
| Aqua | 83.81 |
| Magnesium Chloride | 7.92 |
| Glycerin | 4.94 |
| Cellulose Gum | 1.18 |
| Zeolite* | 2.00 |
| Methylparaben | 0.15 |

| | |
|---|---|
| *zeolite stands for clinoptilolite | |

The water was heated to 75 °C, methlyparaben is dissolved in the water. The mixture is cooled to room temperature, and magnesium chloride was added. The zeolite was suspended in the glycerol, and this suspension was added to the aqueous solution of the magnesium chloride under stirring. Cellulose gum was added and the mixture was stirred until formation of the final gel structure.

### Example 5: Gel formulation

A gel containing the composition of the present invention was formulated with the following ingredients:

| **Ingredient** | **m/m %** |
|---|---|
| Aqua | 77.12 |
| Magnesium Chloride | 7.92 |
| Propylenglycol | 9.90 |
| Hydroxypropyl Cellulose | 1.97 |
| Zeolite* | 2.00 |
| Alcoholum 96% | 0.99 |
| Methylparaben | 0.10 |

| | |
|---|---|
| *zeolite stands for clinoptilolite | |

Magnesium chloride was dissolved in the water. The zeolite was suspended in the propylenglycol, and added under continuous stirring to the aqueous phase. Hydroxyprpyl cellulose was added to the aqueous phase until formation of the final gel structure. Finally, the methylparaben dissolved in the ethanol was added slowly into the gel.

### Example 6: Gel formulation

A gel containing the composition of the present invention was formulated with the following ingredients:

| **Ingredient** | **m/m %** |
|---|---|
| Aqua | 71.92 |
| Magnesium Chloride | 7.83 |
| Glycerol | 2.94 |
| Propylenglycol | 11.76 |
| Cellulose Gum | 0.49 |
| Methylcellulose | 1.96 |
| Zeolite* | 3.00 |
| Methylparaben | 0.10 |

| | |
|---|---|
| *zeolite stands for clinoptilolite | |

The water was heated to 75 °C, methlyparaben is dissolved in the water. The mixture is cooled to room temperature, and magnesium chloride was added. The zeolite was suspended in the glycerol, and this suspension was added to the aqueous solution of the magnesium chloride under stirring. Cellulose gum and methylcellulose was added and the mixture was stirred until formation of the final gel structure.

### Example 7: Cream formulation

A cream containing the composition of the present invention was formulated with the following ingredients:

| **Ingredient** | **m/m %** |
|---|---|
| Aqua | 45.14 |
| Magnesium Chloride | 18.61 |
| White petrolatum | 15.39 |
| Cetearyl alcohol | 9.24 |
| Zeolite* | 4.00 |
| Paraffin | 3.00 |
| Polysorbate 60 | 3.08 |
| Alcohol | 1.39 |
| Methylparaben | 0.15 |

| | |
|---|---|
| *zeolite stands for clinoptilolite | |

Water was heated to 75 °C and magnesium chloride was dissolved in it, followed by dispersion of the zeolite. White petrolatum, cetearyl alcohol, paraffin and polysorbate 60 were mixed in a separate flask and heated to 75 °C. Oil phase was poured to the water phase, emulsified, cooled under continuous stirring, and finally methylparaben dissolved in alcohol was added.

### Example 8: Cream formulation

A cream containing the composition of the present invention was formulated with the following ingredients:

| **Ingredient** | **m/m %** |
|---|---|
| Aqua | 49.25 |
| Magnesium Chloride | 18.61 |
| White petrolatum | 15.39 |
| Cetearyl alcohol | 9.24 |
| Zeolite* | 1.20 |
| Isopropyl miristate | 3.08 |
| Polysorbate 60 | 3.08 |
| Methylparaben | 0.10 |
| Propylparaben | 0.05 |

| | |
|---|---|
| *zeolite stands for clinoptilolite | |

Water was heated to 75 °C and methylparaben and propylparaben was dissolved in it, and zeolite was dispersed in this solution. White petrolatum, cetearyl alcohol, isopropyl myristate and polysorbate 60 were mixed in a separate flask and heated to 75 °C. Oil phase was poured to the water phase, emulsified, cooled under continuous stirring, and finally magnesium chloride dissolved in some part of the water was added.

## Claims

1. Composition containing
a) clay material with a negative surface charge;
b) magnesium ions either incorporated within the clay material and/or added separately;
c) water;
d) substantially non-cationic carrier;
where the total molar amount of magnesium ions compared to calcium ions is higher than 40% of the total molar amount of calcium ions.

2. Composition according to claim 1, where the clay material is a zeolite, more preferably clinoptilolite.

3. Composition according to claim 1 or 2, where the total molar amount of magnesium ions compared to calcium ions is higher than 90% of the total molar amount of calcium ions.

4. Composition according to any of claims from 1 to 3, where the mean particle size of the clay material is between 1 nm and 100 µm, preferably between 50 nm and 500 nm.

5. Composition according to any of claims from 1 to 4, where the separately added magnesium ions are in the form of magnesium chloride or magnesium bromide, preferably magnesium chloride.

6. Use of the composition of any of claims from 1 to 5 for the preparation of a medicament/medical device for the treatment of skin diseases or skin conditions, where the formation and/or recovery of epidermal barrier function has beneficial effects.

7. Use of a composition according to claim 6 where the skin diseases or skin conditions are selected from inflammatory skin diseases, increased fibroblast proliferation, pruritus, physical damage of the skin surface, xerosis, bruises, hyperproliferation states of the skin, and transepidermal water loss.

8. Use of a composition according to claim 7, where the inflammatory skin disease is psoriasis, atopic dermatitis, or various types of eczema.

9. Use of a composition according to any of claims from 6 to 8, where the medicament is in the form of a cream, lotion, gel, ointment, cutaneous solution, suspension, spray, foam, bath additive, collodion, impregnated dressing or medicated plaster.
